# EUROPEAN PATENT APPLICATION

(11) **EP 4 693 316 A1**
(43) Date of publication of application: **11.02.2026**
(21) Application number: 24778634.6
(22) Date of filing: 29.01.2024
(51) Int. Cl.: G16H 10/00

(54) **LEARNING MODEL CREATION DEVICE AND LEARNING MODEL CREATION METHOD**

(30) Priority: 27.03.2023 JP 2023049847
(71) Applicant: FUJIFILM Corporation, Tokyo 106-8620 (JP)
(72) Inventor: ITO, Atsushi, Ashigarakami-gun, Kanagawa 258-8577 (JP)
(74) Representative: Dehns Germany Partnerschaft mbB
(86) International application number: PCT/JP2024/002574
(87) International publication number: WO 2024/202488

(57) **Abstract**

Provided are a learning model creation device and a learning model creation method that can grasp a usage aspect of data. A processor (40) receives a learning request and starts a process of creating a learning model. In a case where the process is started, the processor (40) reads out medical data corresponding to a range designated in the learning request from a medical data management server (22). Then, the processor (40) creates the learning model using the read out medical data and a learning algorithm designated in the learning request.

## Description

### BACKGROUND OF THE INVENTION

### 1. Field of the Invention

The present invention relates to a learning model creation device and a learning model creation method.

### 2. Description of the Related Art

In various fields such as the medical field, the creation of learning models though machine learning is widely practiced. As is well known, in a case of creating a learning model though machine learning, a more accurate learning model can be created by performing machine learning (hereinafter, may be simply referred to as learning) using a larger amount of data.

JP6830719B discloses a configuration in which medical data is provided to obtain a consideration, the data provided in this way is used to increase an amount of data used for learning, and thus a more accurate learning model can be created.

### SUMMARY OF THE INVENTION

However, one data (medical data) provided in JP6830719B can be used in a plurality of times of machine learning (to create a plurality of learning models). That is, for example, the data of men aged 80 years can be used for creation of a plurality of learning models such as a learning model for only men, a learning model for adult men, a learning model for elderly men, a learning model for adults regardless of gender, and a learning model for elderly people regardless of gender. In addition, the data includes various types of information (for example, in a case where the data is medical data, height, weight, blood pressure, pre-existing disease, and past medical history) in addition to the age and gender described above, and the number of learning models that can be created using this data also increases as the number of types of information included in the data increases. Therefore, in a case where the medical data is provided once, there is a problem in that it is not possible to grasp a usage aspect of the provided data (such as which learning model the data is used to create and how many times the data is used).

The present invention has been made in view of the above background, and an object of the present invention is to provide a learning model creation device and a learning model creation method that can grasp a usage aspect of data.

In order to achieve the above-described object, an aspect of the present invention relates to a learning model creation device that creates a learning model through machine learning using data stored in a data management server, the learning model creation device comprising: a learning request reception unit that receives a learning request which is transmitted from a learning request source and in which a learning algorithm used for creating the learning model and a range of the data used for creating the learning model are designated; a data readout unit that reads out the data in the range designated in the learning request from the data management server; and a learning model creation unit that creates the learning model using the data read out from the data management server and the learning algorithm designated in the learning request.

The data may be medical data.

The learning model creation device may further comprise: a learning model transmission unit that transmits the created learning model to the learning request source.

The learning model creation device may further comprise: a learning process preservation unit that stores a learning process including identification information for identifying the data used for creating the learning model on a blockchain.

The learning model creation device may further comprise: a consideration calculation unit that calculates a consideration for use of the data used for creating the learning model.

The learning model creation unit may create a plurality of the learning models through a plurality of times of machine learning using data of a plurality of groups that partially overlap each other.

The consideration calculation unit may calculate, for the data used redundantly in the plurality of times of machine learning, the consideration for the use in accordance with the number of times of redundant use.

The learning model creation unit may create one global learning model by setting each of the plurality of learning models as a local learning model and integrating the local learning models.

In the learning request source, one global learning model may be created by setting each of the plurality of learning models as a local learning model and integrating the local learning models.

In order to achieve the above-described object, another aspect of the present invention relates to a learning model creation method of creating a learning model through machine learning using data stored in a data management server, the learning model creation method comprising: a learning request reception step of receiving a learning request which is transmitted from a learning request source and in which a learning algorithm used for creating the learning model and a range of the data used for creating the learning model are designated; a data readout step of reading out the data in the range designated in the learning request from the data management server; and a learning model creation step of creating the learning model using the data read out from the data management server and the learning algorithm designated in the learning request.

According to the aspects of the present invention, it is possible to provide the learning model creation device and the learning model creation method that can grasp the usage aspect of the data.

### BRIEF DESCRIPTION OF THE DRAWINGS

FIG. 1 is a diagram schematically showing a configuration of a learning system.
FIG. 2 is an explanatory diagram of medical data.
FIG. 3 is an explanatory diagram showing functions of a processor.
FIG. 4 is a flowchart showing a flow of a process of a processor.
FIG. 5 is an explanatory diagram showing functions of the processor.
FIG. 6 is an explanatory diagram showing functions of the processor.

### DESCRIPTION OF THE PREFERRED EMBODIMENTS

### [First Embodiment]

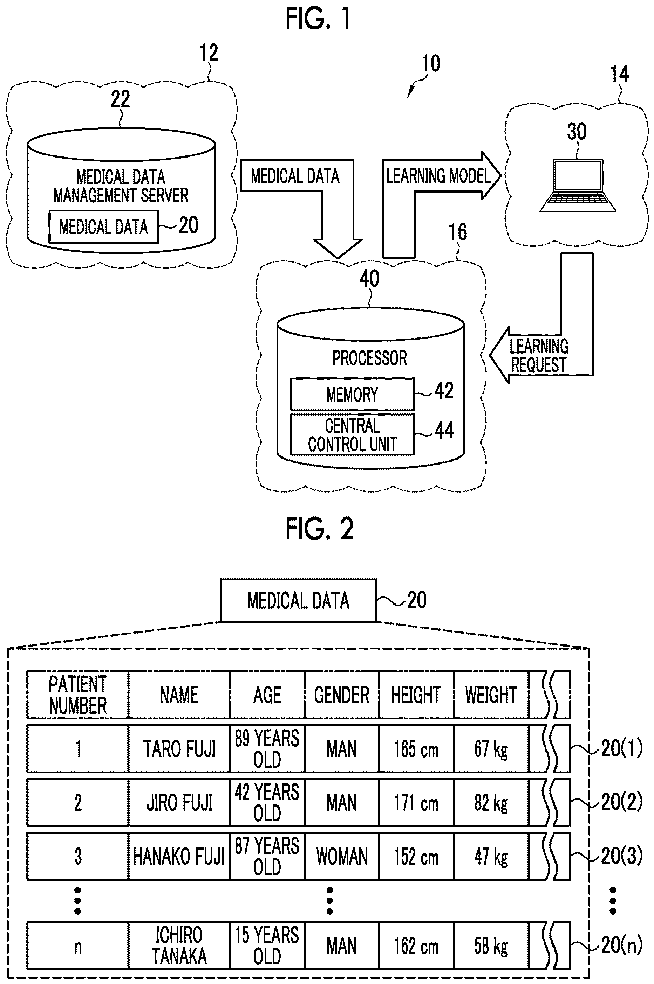

In FIG. 1, a learning system 10 includes a medical data provider environment 12, a learning request source environment 14 (learning request source), and a learning environment 16.

The medical data provider environment 12 is, for example, an environment composed of a medical institution such as a hospital or a part thereof, and comprises a medical data management server 22 (data management server) that stores medical data 20 (data). The medical data 20 is information related to medical care acquired by the medical institution in a medical examination or the like, and is, for example, a result of a health checkup.

As shown in FIG. 2, in the present embodiment, n pieces of medical data 20(1) to 20(n) corresponding to n patients having patient numbers 1 to n (n is any integer) are stored in the medical data management server 22 as the medical data 20. Each of the medical data 20(1) to 20(n) includes information such as name, age, gender, height, and weight, in addition to the patient number described above. In the present embodiment, although an example will be described in which the data is the medical data, the present invention is not limited to this. The data may be data other than the medical data.

Returning to FIG. 1, the learning request source environment 14 is, for example, an environment composed of a medical manufacturer that develops or manufactures a pharmaceutical product or a medical device, or a part thereof, and requests learning (makes a learning request) to the learning environment 16 for the purpose of developing or improving a product. The learning request source environment 14 comprises a request source terminal 30, and the learning request is made via the request source terminal 30. In a case of making the learning request, a type of a learning algorithm (for example, deep learning using a neural network) used for learning and a range of the medical data (for example, men aged 80 years or older) used for learning are designated on the request source terminal 30. In this way, the learning request is a request in which the learning algorithm (type thereof) used for learning and the range of the medical data used for learning are designated.

The learning environment 16 is an environment in which the learning model is generated though machine learning using the medical data, and comprises a processor 40 (learning model creation device). The processor 40 is provided with a memory 42 and a central control unit 44. The memory 42 stores a plurality of learning programs corresponding to various learning algorithms used in machine learning and a control program for causing the processor 40 (central control unit 44) to function as the learning model creation device. The central control unit 44 reads out the control program from the memory 42 in association with the activation of the processor 40, and executes the control program.

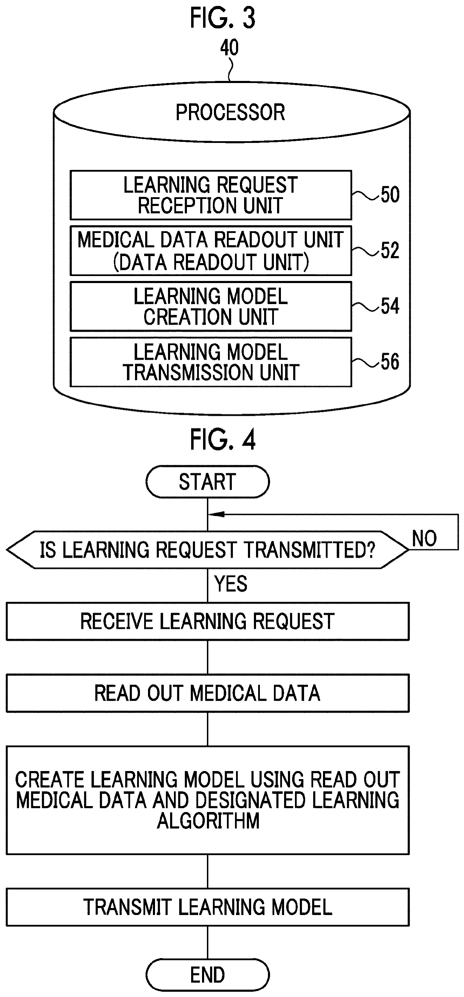

As shown in FIG. 3, in a case where the control program is executed, the processor 40 (central control unit 44) functions as a learning request reception unit 50, a medical data readout unit 52 (data readout unit), a learning model creation unit 54, and a learning model transmission unit 56.

The learning request reception unit 50 receives the learning request transmitted from the learning request source environment 14 (see FIG. 1). As described above, the learning request is a request in which the learning algorithm (type thereof) used for learning and the range of the medical data used for learning are designated. The learning request is transmitted to the processor 40 via the request source terminal 30. The learning request reception unit 50 accepts (receives) the learning request transmitted in this way. As described above, the learning request reception unit 50 executes a learning request reception step according to the embodiment of the present invention.

The medical data readout unit 52 accesses the medical data management server 22 to read out the medical data corresponding to the range designated in the learning request (see FIG. 1). For example, in a case where the range of the medical data designated in the learning request is men aged 80 years or older, in the present embodiment, as shown in FIG. 2, the medical data 20(1) corresponds to the medical data in the designated range. Therefore, the medical data readout unit 52 reads out the medical data 20(1) from the medical data management server 22. As described above, the medical data readout unit 52 executes a data readout step according to the embodiment of the present invention.

The learning model creation unit 54 performs machine learning using the medical data read out by the medical data readout unit 52 to create the learning model. In addition, the learning model creation unit 54 performs machine learning using the learning algorithm designated in the learning request (the learning program for performing learning using the learning algorithm designated in the learning request) to create the learning model. For example, in a case where the learning algorithm designated in the learning request is deep learning using a neural network, the learning model is created by using the learning program for performing learning with the learning algorithm. As described above, the learning model creation unit 54 creates the learning model by using the medical data read out by the medical data readout unit 52 and the learning algorithm designated in the learning request. That is, the learning model creation unit 54 executes a learning model creation step according to the embodiment of the present invention.

The learning model transmission unit 56 transmits the learning model created by the learning model creation unit 54 to the learning request source environment 14 (the request source terminal 30 in the present embodiment) (see FIG. 1).

Hereinafter, a flow of a process (learning model creation method) performed by the processor 40 will be described with reference to FIG. 4. As shown in FIG. 4, in a case where the learning request is transmitted from the learning request source environment 14, the processor 40 receives the learning request (learning request reception step) and starts a process of creating the learning model. In a case where the process is started, the processor 40 reads out the medical data corresponding to the range designated in the learning request from the medical data management server 22 (data readout step). Then, the processor 40 creates the learning model using the read out medical data and the learning algorithm designated in the learning request (learning model creation step), and transmits the created learning model to the request source terminal 30.

As described above, according to the embodiment of the present invention, since the learning model desired by the learning request source environment 14 is created without providing the medical data to the learning request source environment 14 (request source terminal 30), the learning request source environment 14 can grasp the usage aspect of the medical data. In addition, according to the embodiment of the present invention, it is possible to contribute to appropriate management of the medical data. That is, in a case where the medical data is provided to the learning request source environment 14, the medical data may be used in a manner unintended by a provider in the learning request source environment 14, but such a problem can be prevented according to the embodiment of the present invention. Further, in a case where the medical data is provided to the learning request source environment 14, there is a problem in that information not involved in the creation of the learning model is disclosed to the learning request source environment 14. That is, for example, even in a case where only the information on the age and the past medical history is required for the creation of the learning model, information such as the name, the height, and the weight is also disclosed to the learning request source environment 14. According to the embodiment of the present invention, it is possible to prevent the disclosure of such unnecessary information.

### [Second Embodiment]

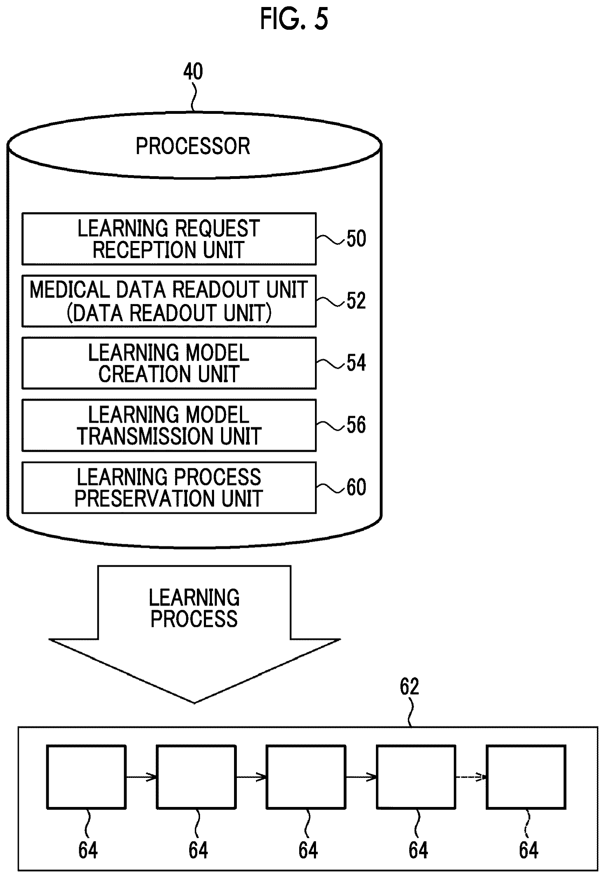

As shown in FIG. 5, in a second embodiment, the processor 40 also functions as a learning process preservation unit 60 in addition to each unit described above. In the description using the drawings from FIG. 5, the same members as the members in the first embodiment described above are denoted by the same reference numerals, and the description thereof will be omitted.

In a case where the learning model is created, the learning process preservation unit 60 preserves a process of creating the learning model (learning process). Specifically, information (hereinafter, process information) necessary for grasping, checking, or the like of the learning process (that is, a process of creating the learning model) is preserved. The process information includes at least identification information for identifying the medical data used for creating the learning model.

The learning process preservation unit 60 preserves the process information by storing the process information on a blockchain 62. The blockchain 62 is constructed by a blockchain network composed of the medical data provider environment 12 (medical data management server 22), the learning request source environment 14 (request source terminal 30), the learning environment 16 (processor 40), and the like, and is obtained by connecting blocks 64 including the process information, in time series. The learning process preservation unit 60 creates a new block 64 each time the learning model is created, and connects the new block 64 to the blockchain 62. In this way, it is possible to prevent the tampering of the learning process by storing the process information on the blockchain 62.

### [Third Embodiment]

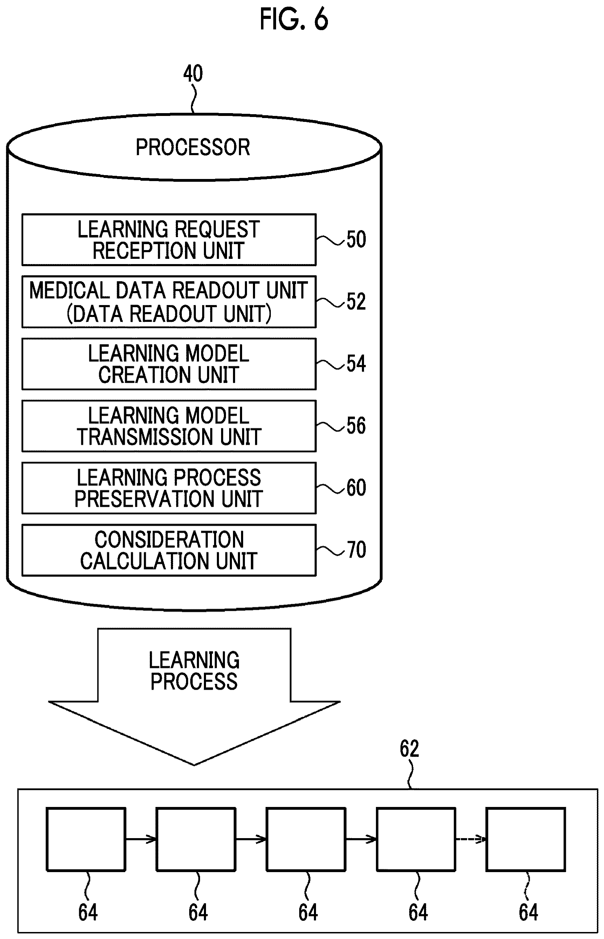

As shown in FIG. 6, in a third embodiment, the processor 40 also functions as a consideration calculation unit 70 in addition to each unit described above. In a case where the learning model is created, the consideration calculation unit 70 calculates a consideration for the use of the medical data 20 used for creating the learning model. The consideration for the use is individually calculated for each of the medical data 20(1) to 20(n), and the consideration for the use is calculated as an amount (value) corresponding to the number of times of use (the number of times of redundant use) of the medical data 20(1) to 20(n), for example, 10 yen per use.

For example, in a case of creating the learning model for men aged 80 years or older, the medical data 20(1) (see FIG. 2) is used once. Therefore, the consideration for the use for one time is calculated for the medical data 20(1) as the consideration for the use. In addition, for example, in a case of creating the learning model for people aged 80 years or older regardless of gender, both the medical data 20(1) and the medical data 20(3) (see FIG. 2) are used once. Therefore, the consideration for the use for one time is calculated for the medical data 20(1) and the medical data 20(3) as the consideration for the use. Further, in a case of creating the two learning models described above, that is, in a case of creating the two learning models, the learning model of men aged 80 years or older and the learning model of people aged 80 years or older regardless of the gender, the medical data 20(1) is used twice, and the medical data 20(3) is used once. Therefore, as the consideration for the use, the consideration for the use for two times is calculated for the medical data 20(1), and the consideration for the use for one time is calculated for the medical data 20(3).

As described above, according to the third embodiment, an appropriate consideration in accordance with the number of times of use can be calculated for each of the medical data 20(1) to 20(n). Then, it is possible to pay such an appropriate consideration to the provider (medical data provider environment 12) or to bill a user (learning request source environment 14).

In the above-described embodiments, the example has been described in which the learning model is created from the medical data, but it is also possible to create a new learning model by integrating the learning models, for example, it is possible to create a learning model of an adult regardless of gender by integrating a learning model of adult men and a learning model of adult women, and it is possible to create a learning model of the entire Japan by integrating a learning model of Kanto (created from the medical data of patients living in Kanto) and a learning model of Kansai. As described above, a configuration may be adopted in which one global learning model is created by setting each of the plurality of learning models as a local learning model and integrating the local learning models.

As described above, in a case where the local learning models are integrated to create the global learning model, the learning environment 16, specifically, the learning model creation unit 54 need only be configured to create the global learning model. It goes without saying that a configuration may be adopted in which the learning request source environment 14 (for example, the request source terminal 30) creates the global learning model.

In addition, in the above-described embodiments, the example has been described in which the processor 40 comprises the plurality of learning programs for performing learning using various learning algorithms, but the present invention is not limited to this. The learning request source environment 14 (for example, the request source terminal 30) may comprise the plurality of learning programs. In this case, the request source terminal 30 need only be configured to transmit the learning program to be used for learning to the processor 40 together with the learning request.

Further, in the above-described embodiments, the example has been described in which the medical data management server 22 is provided separately from the processor 40, but the present invention is not limited to this. The medical data management server 22 may be provided integrally with the processor 40. In such a case, for example, it is conceivable to store the medical data 20 in the memory 42.

In the above-described embodiments, the hardware structures of processing units that execute various types of processing, such as the processor 40, the central control unit 44, the learning request reception unit 50, the medical data readout unit 52, the learning model creation unit 54, the learning model transmission unit 56, the learning process preservation unit 60, and the consideration calculation unit 70, are various processors as described below. The various processors include a central processing unit (CPU) that is a general-purpose processor that functions as various processing units by executing software (programs), a programmable logic device (PLD) that is a processor of which a circuit configuration can be changed after manufacturing, such as a field programmable gate array (FPGA), a dedicated electrical circuit that is a processor of which a circuit configuration is exclusively designed to execute various processes, and the like.

One processing unit may be configured by one of these various processors, or may be configured by a combination of two or more same type or different type of processors (for example, a plurality of FPGAs, or a combination of a CPU and an FPGA). A plurality of processing units may be configured by one processor. As an example in which the plurality of processing units are configured by one processor, first, there is a form in which one processor is configured by a combination of one or more CPUs and software, and this processor functions as the plurality of processing units, as represented by a computer, such as a client or a server. Second, there is a form in which a processor, which implements the functions of the entire system including the plurality of processing units with one integrated circuit (IC) chip, is used, as represented by a system-on-chip (SoC) or the like. As described above, various processing units are configured by one or more of the various processors described above, as the hardware structure.

Further, the hardware structure of these various processors is, more specifically, an electric circuit (circuitry) having a form in which circuit elements, such as semiconductor elements, are combined.

### Explanation of References

10: learning system
12: medical data provider environment
14: learning request source environment
16: learning environment
20: medical data (data)
22: medical data management server (data management server)
30: request source terminal
40: processor (learning model creation device)
42: memory
44: central control unit
50: learning request reception unit
52: medical data readout unit (data readout unit)
54: learning model creation unit
56: learning model transmission unit
60: learning process preservation unit
62: blockchain
64: block
70: consideration calculation unit

## Claims

1. A learning model creation device that creates a learning model through machine learning using data stored in a data management server, the learning model creation device comprising:
a learning request reception unit that receives a learning request which is transmitted from a learning request source and in which a learning algorithm used for creating the learning model and a range of the data used for creating the learning model are designated;
a data readout unit that reads out the data in the range designated in the learning request from the data management server; and
a learning model creation unit that creates the learning model using the data read out from the data management server and the learning algorithm designated in the learning request.

2. The learning model creation device according to claim 1,
wherein the data is medical data.

3. The learning model creation device according to claim 2, further comprising:
a learning model transmission unit that transmits the created learning model to the learning request source.

4. The learning model creation device according to claim 3, further comprising:
a learning process preservation unit that stores a learning process including identification information for identifying the data used for creating the learning model on a blockchain.

5. The learning model creation device according to claim 4, further comprising:
a consideration calculation unit that calculates a consideration for use of the data used for creating the learning model.

6. The learning model creation device according to claim 5,
wherein the learning model creation unit creates a plurality of the learning models through a plurality of times of machine learning using data of a plurality of groups that partially overlap each other.

7. The learning model creation device according to claim 6,
wherein the consideration calculation unit calculates, for the data used redundantly in the plurality of times of machine learning, the consideration for the use in accordance with the number of times of redundant use.

8. The learning model creation device according to claim 6 or 7,
wherein the learning model creation unit creates one global learning model by setting each of the plurality of learning models as a local learning model and integrating the local learning models.

9. The learning model creation device according to claim 6 or 7,
wherein, in the learning request source, one global learning model is created by setting each of the plurality of learning models as a local learning model and integrating the local learning models.

10. A learning model creation method of creating a learning model through machine learning using data stored in a data management server, the learning model creation method comprising:
a learning request reception step of receiving a learning request which is transmitted from a learning request source and in which a learning algorithm used for creating the learning model and a range of the data used for creating the learning model are designated;
a data readout step of reading out the data in the range designated in the learning request from the data management server; and
a learning model creation step of creating the learning model using the data read out from the data management server and the learning algorithm designated in the learning request.
